# EUROPÄISCHE PATENTSCHRIFT

(11) **EP 2 523 713 B1**
(45) Veröffentlichungstag und Bekanntmachung des Hinweises auf die Patenterteilung: **26.08.2015**
(21) Anmeldenummer: 11700255.0
(22) Anmeldetag: 10.01.2011
(51) Int. Cl.: A61M 15/06

(54) **INHALATORSYSTEM FÜR FLÜCHTIGE SUBSTANZEN**
INHALER SYSTEM FOR VOLATILE SUBSTANCES
SYSTÈME D'INHALATEUR POUR SUBSTANCES VOLATILES

(30) Priorität: 11.01.2010 DE 102010000043
(43) Veröffentlichungstag der Anmeldung: 21.11.2012
(73) Patentinhaber: Surflay Nanotec GmbH, 12489 Berlin (DE)
(72) Erfinder: DÄHNE, Lars, 12587 Berlin (DE); EGRI, Gabriella, 12524 Berlin (DE); ALDENHOVEN, Claudia, 10967 Berlin (DE)
(74) Vertreter: Zimmermann & Partner Patentanwälte mbB
(86) Internationale Anmeldenummer: PCT/EP2011/050232
(87) Internationale Veröffentlichungsnummer: WO 2011/083168

(56) Entgegenhaltungen:
- WO-A2-2008/113335
- DE-A1-102004 013 637
- DE-U1-202007 004 678

## Beschreibung

Die vorliegende Offenbarung betrifft ein Depot zur Speicherung von flüchtigen Substanzen, beispielsweise Wirk- und Aromastoffen, ein Inhalatorsystem für die Freisetzung flüchtiger Wirk- und Aromastoffe aus diesem Depot, sowie die Verwendung des Depots, beispielsweise zur Aufnahme bzw. Verabreichung von Genussmitteln, das Wohlbefinden steigernden Substanzen, pharmazeutischen Wirkstoffen, Pestiziden oder Aromastoffen.

Aus einem Filter mit einem Nikotin-Depot bestehende und Zigaretten ähnelnde. Vorrichtungen werden in WO 2004/098324, WO 2004/095955, WO 2009/105919, DE 10 2008 014 587, DE 10 2005 054 255 A1, DE 103 56 925 B4, WO 2006/002 445 A2 und AT 505 472 A1 beschrieben.

Aus der WO2008/113335 A2 ist ein Depot filder aus Polyethylen-Fasern mit einer inneren Bohrung bekannt.

Vor diesem Hintergrund wird ein Depot nach Anspruch 1 oder 3 vorgeschlagen. Weiterhin wird eine Kartusche für eine Dosiervorrichtung nach Anspruch 8 vorgeschlagen. Weiterhin wird eine rauchfreie Zigarette, Zigarillo, Zigarre oder Tabakspfeife nach Anspruch 9 vorgeschlagen. Weiterhin wird eine Vorrichtung zur diskontinuierlichen oder kontinuierlichen Freisetzung einer Substanz mit einem Gasstrom nach Anspruch 11 vorgeschlagen. Weiterhin wird die Verwendung eines Depots oder einer Kartusche vorgeschlagen. Weiterhin werden Verfahren zur Herstellung eines Depots vorgeschlagen. - Weitere Ausführungsformen und Vorteile ergeben sich aus den Unteransprüchen, den nachfolgenden Erläuterungen und den Figuren.

Die vorliegende Erfindung wird beispielhaft an Hand von Figuren erläutert. Dabei zeigt
- Fig. 1: den schematischen Aufbau eines Depots (Hybriddepot);
- Fig. 2: die Adsorption von Rhodamin-Polyallylamin in nanoporösen Partikeln;
- Fig. 3: die spektroskopische Analyse von Nikotin nach Beispiel 1;
- Fig. 4: die Mikroskopie-Aufnahme eines makroporösen Depots (Beispiel 2);
- Fig. 5: die Nikotinfreisetzung aus dem Depot nach Beispiel 5;
- Fig. 6: die Nikotinfreisetzung aus dem Depot nach Beispiel 6;
- Fig. 7: den Vergleich von Depots nach Herstellung und Lagerung (Beispiel 7);
- Fig. 8: die Nikotinfreisetzung aus einem Depot nach Beispiel 9;
- Fig. 9: die Nikotinfreisetzung aus einem Depot nach Beispiel 10.

Bei der Suche nach einem Depot für Wirk- oder Aromastoffe für deren Freisetzung in einen Gasstrom wurde überraschend gefunden, dass sich Hybridmaterialien einer Kombination aus einem makroporösen Stützgerüst mit daran dauerhaft befestigten nanoporösen Speicherpartikeln dafür gut eignen.

Technische Vorteile der hier beschriebenen Ausführungsformen bestehen in der Bereitstellung eines filterartigen Wirkstoff-Depots für ein Inhalatorsystem, zum Beispiel für eine rauchfreie Zigarette, ein rauchfreies Zigarillo, eine rauchfreie Zigarre oder eine rauchfreie Tabakspfeife und für die inhalative Verabreichung von das Wohlbefinden steigernden Substanzen oder Substanzgemischen. Der Luftwiderstand des beispielsweise filterartigen Wirkstoffdepots kann dabei gering sein, ohne dass, außer den gewünschten Wirk-und Aromastoffen, andere Substanzen oder Partikel freigesetzt werden. Im Folgenden soll der Begriff rauchfreie Zigarette auch ein rauchfreies Zigarillo, eine rauchfreie Zigarre oder eine rauchfreie Tabakspfeife umfassen. Rauchfrei soll hier bedeuten, dass keine Verbrennung von Tabak oder ähnlichen Rauchwaren erfolgt.

Weitere technische Vorteile beschriebener Ausführungsformen bestehen in der Bereitstellung eines Dosiersystems, zum Beispiel in Form eines Depots, für die geregelte Freisetzung von Wirk- und/oder Aromastoffen in die Luft, beispielsweise in die Luft geschlossener Räume. Die im Wirkstoffdepot gespeicherten Stoffe können beim Durchströmen mit einem Gas oder Gasgemisch über einen vorgegebenen Zeitraum in vorgegebenen Mengen freigesetzt werden.

Als geschlossene Räume kommen insbesondere Lagerräume, beispielsweise zur geschützten Aufbewahrung von Museumsstücken; Speicher und Silozellen zur Lagerung von Ernteprodukten, Saat- und Pflanzgut (Vorratsschutz); Gewächshäuser, beispielsweise für den Zierpflanzen- und Gemüseanbau; andere landwirtschaftliche Lagerräume und Lagerräume der Lebensmittelindustrie oder auch kleinere abgeschlossene Kammern und Behältnisse mit geregelter Luftfeuchtigkeit oder/und geregelter Temperatur in Betracht. Die für diese Anwendungen in Betracht kommenden Substanzen sind typischerweise biologisch aktive Wirkstoffe oder Wirkstoffgemische, beispielsweise mit insektizider oder akarizider Wirkung. Beispiele für derartige Substanzen sind Cinerin, Jasmolin oder Pyrethrin, auch andere Pyrethroide. Diese Substanzen enthaltende Depots können vorteilhafterweise direkt mit synergistisch wirkenden Substanzen, beispielsweise mit Piperonylbutoxyd, oder diese Synergisten enthaltenden Depots kombiniert werden. Weitere Beispiele für Wirkstoffe sind p-Menthan-diol. Auch Rodentizide können als Substanzen im Depot eingebracht, bzw. vom Depot gezielt freigesetzt werden.

Weiterhin sind Anwendungen für Räume mit besonderen hygienischen Anforderungen an Reinheit oder Duft, wie beispielweise (öffentliche) Toiletten, Bäder, Schwimmbäder, Duschräume, Sport- und Trainings-Räume, Fitness-Center, Sauna etc. möglich.

Nach einer oder mehreren Ausführungsformen kann das Depot direkt oder als Bestandteil eines Dosiersystems für die lokale Freisetzung von Substanzen im Freien genutzt werden. Eine hierfür typische Anwendung kann die Freisetzung von Repellents sein, beispielsweise im Forstschutz zur Wildschadenverhütung. Der im Depot gespeicherte Wirkstoff oder ein Substanz- Gemisch entfaltet mit seiner Freisetzung beispielsweise eine vergrämende Wirkung für schadenverursachende Wildtierarten in der freien Natur, in Forstanlagen, Jungpflanzungen, Baumschulen, oder Garten- und Parkanlagen. Nach einer oder mehreren anderen Ausführungsformen kann das Depot bzw. das Dosiersystem mit diesen Repellents zur Vergrämung von Haustieren von Gebäuden oder Flächen, beispielsweise entlang von Grundstücksgrenzen Verwendung finden.

Eine oder mehrere weitere Ausführungsformen betreffen die Verwendung des Depots zur Freisetzung von Insekten-Repellents zum Schutz vor lästigen Insekten, beispielsweise in Räumen oder auf Freiflächen oder Gartenanlagen. Eine oder mehrere weitere Ausführungsformen betreffen die gezielte Freisetzung von Pheromonen zur gezielten Anwendung im integrierten Pflanzenschutz vor Schadinsekten, beispielsweise mit Pheromonfallen.

Eine oder mehrere Ausführungsformen umfassen ein Hybridmaterial für ein Wirkstoffdepot, welches unterschiedliche Funktionen in einem Filtersystem vereint. Beispielsweise wird ein offenporiges Material mit Porenweiten im Mikrometerbereich (auch Makroporen genannt), das Luft ohne großen Widerstand passieren lässt, mit nanoporösen Partikeln kombiniert. Weiterhin können die nanoporösen Partikel an der Oberfläche eines offenporigen Stützgerüsts dauerhaft fixiert sein. Die Menge und die Größe der nanoporösen Partikel sind so gewählt, dass die Makroporen einerseits nicht verstopft werden und andererseits genügend Wirkstoff im Inneren der Partikel adsorbiert werden kann. Die Wirk- und Aromastoffe liegen im Inneren der nanoporösen Partikeln beispielsweise adsorbiert vor. Insbesondere können die Wirk- und Aromastoffe direkt vorliegen, d.h. ohne dass sie in einem Lösungsmittel gelöst sind. Es hat sich gezeigt, dass die lösungsmittelfrei Adsorption für die spätere Abgabe der Wirk- und Aromastoffe förderlich ist. Nach einer oder mehreren Ausführungsformen kann das Stützgerüst aus Partikeln, Fasern, partikulärem Material, oder Gemischen aus diesen Materialien aufgebaut sein. Das partikuläre Material oder Materialgemisch kann beispielsweise gesintert, angeschmolzen, geklebt oder anderweitig untereinander verbunden werden, so dass ein mechanisch stabiles offenporiger Stützgerüst entsteht.

Nach einer oder mehreren Ausführungsformen kann das Stützgerüst auch aus einer losen Schüttung aus partikulärem Material oder einem Materialgemisch bestehen, wobei das Stützgerüsts in einem geeigneten Behälter vorliegt.

Gemäß einer oder mehrerer Ausführungsformen kann die Freisetzung der im Hybridmaterial gebunden oder adsorbiert vorliegenden Substanzen in das anströmende Gas bevorzugt durch Konvektion und/oder Diffusion erfolgen. Die bevorzugten strukturellen Besonderheiten des Depots, beispielsweise des Stützgerüstes können sich aus den für die jeweilige Anwendung jeweils bevorzugten Freisetzungsraten der im Depot vorliegenden Substanz oder der Substanzen ergeben.

Gemäß einer oder mehrerer Ausführungsformen kann ein Depot für die Speicherung und Abgabe einer Substanz in die Gasphase eine Hybridstruktur sein, die gebildet wird aus einem offenporigen makroporösen Stützgerüst mit Kanälen, durch die ein Gas leicht durchströmen kann, und aus immobilisierten, nanoporösen Partikeln, die in nanometergroßen Poren den freizusetzenden Wirkstoff enthalten und welche über die Kanäle des Stützgerüstes miteinander verbunden sind, wobei das Stützgerüst aus dauerhaft miteinander verbundenen Partikeln oder Fasern besteht. Die Kanäle weisen beispielsweise eine mittlere Weite auf, die insgesamt ein Durchströmen des Gases gestatten. Beispielsweise kann die Kanalweite (beispielsweise Durchmesser, sofern die Kanäle einen etwa kreisförmigen Querschnitt haben) im Bereich einiger hundert Mikrometer liegen, beispielsweise zwischen 100µm und 800µm. Es versteht sich jedoch von selbst, dass diese Angabe sich auf eine mittlere Kanalweite bezieht, da die Kanäle eine unregelmäßige Form und auch eine Kanalweitenverteilung aufweisen können.

Gemäß einer oder mehrerer Ausführungsformen können die zur Bildung des Stützgerüsts genutzten Mikropartikel Größen von 200 bis 1000 µm aufweisen. An der Oberfläche der beispielsweise durch das Sintern gebildeten Poren können nanoporöse Partikeln dauerhaft fixiert werden. In einer oder mehreren Ausführungsformen weist das offenporige Stützgerüst Makroporen auf, die größer sind, als die nanoporösen Partikeln, welche zur Aufnahme des Wirkstoffs oder des Aromastoffs dienen. Gemäß einer oder mehrerer Ausführungsformen liegt die Größe der im Stützgerüst vorliegenden Makroporen typischerweise im Bereich zwischen 10 µm und 800 µm, beispielsweise zwischen 20 µm und 700 µm, insbesondere zwischen 25 µm und 600 µm.

Die genannten nanoporösen Partikel weisen gemäß einer oder mehrerer Ausführungsformen typischerweise Partikeldurchmesser von 5 µm bis 500 µm auf, beispielsweise Durchmesser zwischen 10 µm bis 300 µm, insbesondere Durchmesser zwischen 20 µm und 150 µm. Ihre Nanoporen liegen typischerweise im Bereich zwischen 1 nm bis 900 nm, beispielsweise liegen ihre Porendurchmesser im Bereich von 3 nm bis 700 nm und insbesondere zwischen 6 nm bis 500 nm. Im gleichen Größenbereich können auch die im Stützgerüsts ausgebildeten Nanoporen liegen.

Gemäß einer oder mehreren Ausführungsformen werden die nanoporösen Partikel zusammen mit dem partikulären Material (Mikropartikel, Fasern oder dergleichen) gemischt und dann das partikuläre Material miteinander zur Bildung eines offenporigen Stützgerüsts verbunden, wobei dadurch gleichzeitig die nanoporösen Partikel im oder am Stützgerüst, immobilisiert werden.

Gemäß einer oder mehrerer Ausführungsformen weisen die nanoporösen Partikel im Vergleich zum Material, aus dem das Stützgerüst besteht, oder im Vergleich zu den Partikeln, aus denen das Stützgerüst gebildet wird, eine höhere Schmelztemperatur auf. Dadurch wird gewährleistet, dass bei einer thermischen Behandlung zur Verfestigung des Stützgerüsts die nanoporösen Partikel nicht schmelzen oder deren Poren verkleben. Die nanoporösen Partikel können somit in die Oberfläche der Stützgerüstpartikel beim Erweichen "eingebettet" werden.

In verschiedenen Ausführungsformen dienen partikel- oder faserförmige Sintermaterialien aus anorganischen Materialien, wie beispielsweise Silikate oder Alumosilikate, zur Bildung des makroporösen Stützgerüsts des Depots. Ebenso können organische Polymere für das makroporöse Stützgerüst verwendet werden, wie beispielsweise Polyethylen oder Polypropylen, Polyvinylchlorid, Polycarbonat, Polyester und weitere Materialien. Für inhalative Zwecke sollte darauf geachtet werden, dass keine Weichmacher in den Polymeren enthalten sind. Im Fall von partikulären Sintermaterialien sollte deren Partikelgröße so angepasst sein, dass bei der gewünschten Länge des Depots die Luft leicht hindurch strömen kann.

Nach einer oder mehreren Ausführungsformen kann das Depot durch ein Gemisch aus unterschiedlich großen Stützpartikeln oder Fasern bzw. aus Partikeln die aus unterschiedlichen Materialien bestehen, gebildet werden. Ebenso können verschiedene nanoporöse Partikel gleichzeitig in dem Stützgerüst eingebettet werden, die sich in ihrer Größe und/oder Form und/oder Porengröße und/oder Material und/oder Affinität zu dem Wirkstoff unterscheiden. Die zur Herstellung des Depots verwendeten Partikel können ebenso ein Gemisch unterschiedlich beladener oder mit unterschiedlichen Substanzen beladener poröser Partikel sein.

Nach einer oder mehreren Ausführungsformen sind die Größe und die Form des Depots an die jeweilige Anwendung angepasst. Sie ist insbesondere adaptiert an die geometrischen Gegebenheiten des Inhalations- bzw. des Dosiersystems. Beispielsweise kann das Depot als Kartusche für die Unterbringung im Kopf oder Holm einer konventionellen Tabakspfeife gestaltet werden oder die Größe, Form und Farbe eines Zigarettenfilters aufweisen oder zum Einsatz in einem Imitat einer Zigarre oder eines Zigarillos hergerichtet sein. Liegt der Einsatz des Dosiersystems auf einem Gebiet der Landwirtschaft oder Vorratshaltung, so ist das Depot für die jeweilige anwendungsgemäße Freisetzung der jeweiligen Wirkstoffe hergerichtet und angepasst. Mit dieser Anpassung wird eine gute Zugänglichkeit der Nanoporen für den jeweils anströmenden Gas- oder Luftstrom und die gleichmäßige Verteilung der Substanzen darin erreicht. Dabei kann die Form des Depots und/oder der Kartusche vorteilhafterweise zylindrisch aber auch eckig sein, wie beispielsweise kubisch oder quaderförmig. Gemäß einer oder mehrere Ausführungsformen weist ein austauschbarer Filterstick für eine rauchfreie Zigarette das Depot auf. Die rauchfreie Zigarette kann gemäß einer oder mehrere Ausführungsformen ohne zusätzliche Heizquellen gebildet sein. In diesem Fall weist der angesaugte Gas- bzw. Luftstrom in etwa die Temperatur der Umgebung auf. Gemäß einer oder mehrere Ausführungsformen kann die rauchfreie Zigarette eine Heizquelle aufweisen, um den angesaugten Gasstrom oder das Depot zu heizen. Gemäß einer oder mehrere Ausführungsformen kann das Depot in Form einer Filterscheibe oder Filterdepots ausgebildet sein.

Gemäß einer oder mehrer Ausführungsformen wird eine rauchfreie Zigarette, Zigarillo, Zigarre oder Tabakspfeife, aufweisend einen Grundkörper mit einem Behältnis zur Aufnahme eines Depots bereitgestellt, wobei die Substanz Nikotin, ein Nikotinsalz oder ein Inhaltsstoff von Zigarren- oder Zigarettentabak ist, der in den Nanoporen adsorbiert oder gebunden vorliegt.

Beispielsweise kann je nach Verwendungszweck die Höhe (Länge) und Breite (Durchmesser) eines makroporösen zylinderförmigen Stützgerüstes in Abhängigkeit von der Flüchtigkeit des Wirkstoffes, der Temperatur des ankommenden Luftstromes, der gewünschten Konzentration des Wirkstoffes im das Depot verlassenden Gasstrom, der Beladungsmenge, dem Strömungswiderstand, sowie der dafür zu immobilisierenden Menge nanoporöser Partikeln geeignet gewählt werden.

Überraschenderweise eignen sich anorganische Adsorbermaterialien mit einer möglichst großen inneren Oberfläche gut als nanoporöse Depotmaterialien für Wirkstoffe und Aromastoffe. Die innere Oberfläche der Adsorbermaterialien wird von den Wänden der Poren gebildet, so dass eine große Kontaktfläche für porengängige Substanzen zur Verfügung steht. Neben porösen Alumosilikaten eignen sich nanoporöse Silikate besonders gut zur Aufnahme von Wirkstoffen wie beispielsweise p-Menthandiol, Menthol, Salbutamol oder Nikotin.

Diese Adsorbermaterialien weisen ein offenporiges Netz miteinander verbundener Röhren auf. Sie sind mit definierten mittleren Porenweiten, zumeist von 3 nm, 7 nm, 12 nm, 30 nm und 100 nm Porendurchmesser als chromatographische Trägermaterialien kommerziell erhältlich. Möglich sind aber auch breitere Porenverteilungen im Nanometerbereich, wie z.B. von 3 nm bis 700 nm.

Die Partikelgröße geeigneter nanoporöser Materialien liegt beispielsweise bei 10 µm bis 1 mm, insbesondere von 50 µm bis 700 µm. Die nanoporösen Materialien können eine sphärische Form aufweisen. Gemäß einer Ausführungsform werden zur Einbettung irregular gebrochene Materialien und damit irregulär geformte Partikel verwendet.

Gemäß einer oder mehrerer Ausführungsformen ist der Wirk- oder Aromastoff Nikotin oder ein Nikotinderivat. Der Wirkstoff Nikotin stellt eine bei Raumtemperatur farblose, häufig aber durch beginnende Oxidation bereits leicht gelbliche Flüssigkeit dar. Nikotin weist einen relativ hohen Siedepunkt von 246 °C auf, so dass bei niedrigeren Temperaturen die Sättigungskonzentration des Nikotins in Luft stark abnimmt. Nikotin ist anfällig für die Oxidation mit Luftsauerstoff, so dass es sich in Kontakt damit zu einer dunkelbraun gefärbten Verbindung zersetzt. Gemäß einer oder mehrere Ausführungsformen können zwei Depots miteinander kombiniert werden, in denen jeweils unterschiedliche Substanzen adsorbiert sind. Beispielsweise kann eine der Substanzen zur teilweisen oder vollständigen Modifikation, beispielsweise chemischen Umwandlung, der anderen Substanz dienen. Beispielsweise kann eine Substanz Nikotin sein, die durch die andere Substanz, beispielsweise eine Säure, zumindest teilweise in ein Nikotinsalz überführt werden, um die Verträglichkeit des inhalierten Nikotins zu verbessern. Die Säure ist daher in dem "stromaufwärts" gelegenen Depot enthalten. Der Filterstick für die rauchfreie Zigarette, oder auch eine Kartusche für eine Dosiervorrichtung, kann daher mindestens ein Depot aufweisen. Gemäß einer oder mehrere Ausführungsformen können der Filterstick und die Kartusche wenigstens zwei in Gasflussrichtung hintereinander angeordnete Depots aufweisen.

Versuche zur Langzeitstabilität von Nikotin im beschriebenen Depot im Vergleich zu üblicherweise verwendeten Trägermaterialien, wie Acetatfasern, ergaben eine deutlich verringerte Gelb- bzw. Braunfärbung. Das in nanoporösen Partikeln vorliegende Nikotin ist im Vergleich zu Nikotin in einem saugfähigen Träger, beispielsweise Acetatfasern, stabiler gegenüber einer Oxidation an der Luft. Das ist bemerkenswert, da die von den nanoporösen Partikeln bereitgestellte Kontaktfläche zur Luft sehr groß ist. Es wird vermutet, ohne sich darauf einschränken zu wollen, dass sich die Oxidationsempfindlichkeit von Nikotin mit seiner Adsorption an die Oberfläche, beispielsweise die innere und äußere Oberfläche von Silika-Partikeln, des nanoporösen Materials deutlich verringert.

Die nanoporösen Materialien besitzen neben der hohen Adsorptionskapazität für polare Wirkstoffe wie Nikotin, auch eine Affinität zu weniger polaren Stoffen, wie z.B. Aromastoffen oder ätherischen Ölen. Derartige Aromastoffe können separat oder simultan zur jeweiligen Substanz, z.B. Nikotin, adsorbiert und zusätzlich freigesetzt werden. Von besonderem Interesse sind in diesem Zusammenhang Terpene und Terpenoide, insbesondere Mono- und Sesquiterpene sowie ätherische Öle oder Feststoffe wie Latschenkieferöl, Eukalyptusöl, Pfefferminzöl, Nelkenöl, Menthol. Weiterhin besteht eine hohe Adsorptionskapazität zu chemischen oder natürlichen Insektenrepellentien wie z.B. p-Menthane-3,8-diol. Auch Bronchospasmolytika zur Inhalation bei Asthmabeschwerden wie beispielsweise Salbutamol, Formoterol, Theophyllin oder Terbutalin lassen sich in diesen Materialien adsorbieren.

Damit können Kombinationen von Wirkstoffen mit bevorzugten oder für die jeweilige Anwendung wesentlichen Aromen in den Luft- oder Gasstrom abgegeben werden. Sie können beispielsweise dem Inhalat einen charakteristischen Geschmack verleihen oder aber die Luft in einem abgeschlossenen Raum mit einer gewünschten Konzentration der Substanz anreichern.

Nach einer oder mehreren Ausführungsformen können ebenso auch Pestizide, wie beispielsweise Insektizide, Akarizide oder Rodentizide oder biologische Wirkstoffe wie Cinerin, Jasmolin, Pyrethrin, als auch Insektenrepellents wie z.B. p-Menthan-diol, Diethyltoluamid (DEET) oder Permethrin als Wirkstoffe im Depotmaterial gespeichert und mit einem entsprechenden Dosiersystem geregelt freigesetzt werden. Weitere Wirkstoffe zum Anlocken von Insekten umfassen die große Gruppe der Pheromone, die mit dem erfindungsgemäßen System ebenfalls dosiert in die Umgebungsluft abgegeben werden können. Eine oder mehrere weitere Ausführungsformen der Erfindung bestehen darin, verschiedene Wirk-, Aroma- oder Hilfsstoffe, deren Wechselwirkung miteinander zu einem negativen Effekt bei der gemeinsamen Freisetzung führt, in verschiedenen nacheinander geschalteten Hybriddepots unterzubringen, durch welche die Luft strömt.

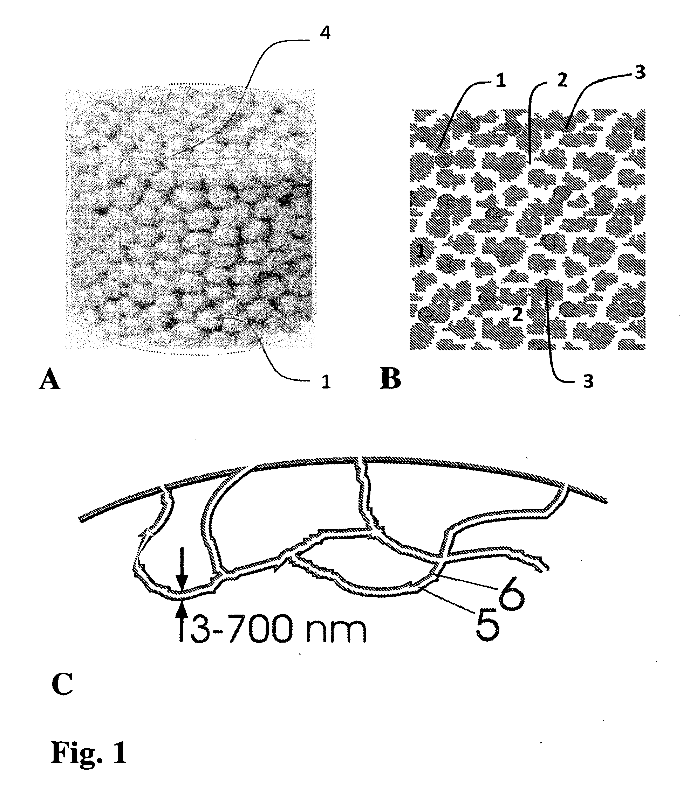

Figur 1 zeigt den schematischen Aufbau des Depots zur Speicherung flüchtiger Wirk- und/oder Aromastoffe: In Figur 1A ist ein aus gesinterten Partikeln (1) gebildetes makroporöses Stützgerüst dargestellt. Das mit einer gestrichelten Linie dargestellte Rechteck (4) stellt schematisch eine Schnittebene durch dieses gesinterte Stützgerüst dar. Figur 1 B zeigt schematisch die vergrößerte Ansicht des in Figur 1A dargestellten Schnitts (4) durch das Stützgerüst. Dabei bezeichnet Bezugszeichen 2 die Poren, Kavernen und Zwischenräume der das Stützgerüst bildenden Partikel (1). Diese Poren, Kavernen und Zwischenräume (2) bilden ein durchgängiges Netzwerk in dem die nanoporösen Partikel (3) stabil und dauerhaft angehaftet bzw. eingebettet sind. In Figur 1C ist schematisch die Detailansicht eines Schnitts durch ein solches nanoporöses Partikel (3) gezeigt. Das nanoporöse Partikel weist Poren (5) in dem Bereich von 1 bis 900 nm auf. An der Oberfläche des nanoporösen Partikels, insbesondere auf der inneren, von den Porenwänden gebildeten Oberfläche des nanoporösen Partikels ist der Wirk- oder Aromastoff (6) adsorbiert und steht dort im ständigen Austausch mit dem durch das Partikel diffundierenden oder strömenden Gas.

Soll ein solches Depot beispielsweise zur rauchfreien Inhalation eines Wirkstoffes wie Nikotin genutzt werden, kann das Depot in Größe, Form und Farbe an ein konventionelles Zigarettenfilter angepasst sein. Genauso kann das Depot als Kartusche für die Unterbringung im Kopf oder Holm einer konventionellen Tabakspfeife gestaltet werden.

Gemäß einer oder mehrerer Ausführungsformen kann das Depot in einer Röhre oder einem Behältnis mit zumindest einer Einlass-Öffnung und zumindest einer AuslassÖffnung, beispielsweise einem Zylinder befestigt sein. Beispielsweise kann das Behältnis angepasst sein, um in einer rauchfreien Zigarette, einem rauchfreien Zigarillo, einer rauchfreien Zigarre oder rauchfreien Tabakspfeife mit der optischen und haptischen Anmutung einer Zigarette, beziehungsweise eines Zigarillo, einer Zigarre oder einer Tabakspfeife, eingesetzt oder befestigt zu werden.

Das hier beschriebene Depot kann dann einerseits eine ausreichende NikotinFreisetzung ein die Atemluft gewährleisten und andererseits das Nikotin vor Oxidation schützen.

Aus dem durchschnittlichen Zugvolumen von 35 ml und der Zugzeit eines durchschnittlichen Rauchers von 1-2 Sekunden ergibt sich eine sehr kurze Kontaktzeit der das Depotfilter durchströmenden Luft mit dem Nikotinreservoir. Der Übergang des Nikotins in die Gasphase korreliert in etwa mit der Kontaktfläche zwischen dem Nikotinreservoir und der Luft.

Die von den nanoporösen Partikeln auf dem mikroporösen Stützgerüst für die durchströmende Luft bereitgestellte Kontaktfläche zum adsorbiertem Wirkstoff ist im Vergleich zur Kontaktfläche der üblicherweise verwendeten flüssigkeitsgefüllten Kapillaren saugfähiger Materialien sehr groß. Während in letzteren als Verdunstungsfläche lediglich die jeweils zugänglichen Querschnitte (Menisken) der gefüllten Kapillaren in Betracht kommen, vollzieht sich der Gasaustausch im Fall des beschriebenen Depots über dessen gesamte Oberfläche, die den adsorbierten Wirkstoff aufweist.

Untersuchungen zur Adsorption von Stickstoffmolekülen an der Oberfläche von derartigen nanoporösen Materialien gemäß der üblichen BET - Methode zeigen, dass ihre innere Oberfläche mehr als 270 m²/g betragen kann. Das ist ein Vielfaches der wirksamen äußeren Oberfläche der beispielsweise in DE 102008014587 A1 angeführten nichtporösen Nanopartikel. Bei einem Durchmesser der Nanopartikel von etwa 100 nm, wie in einer oder mehreren Ausführungsformen der vorstehend genannten Druckschrift verwendet, beträgt die Oberfläche nur 33 m²/g.

Die Fixierung der nanoporösen Partikeln an der Oberfläche des beispielsweise beim Sintern gebildeten makroporösen Stützgerüsts kann so erfolgen, dass die Nanoporen des fixierten Partikels auf seiner von der Oberfläche des Stützgerüsts abgewandten Seite nicht verschlossen werden. Auch beim Befüllen des Depots wird das Verschließen dieser Poren vermieden.

Gemäß einer oder mehreren Ausführungsformen werden die nanoporösen Partikel nicht durch Auftropfen des reinen flüssigen Wirkstoffs, wie z.B. Nikotin, befüllt. Die Befüllung erfolgt nach Auflösen der Wirkstoffe in einem geeigneten, beispielsweise leicht flüchtigen, organischen Lösungsmittel, das nach vollständigem Verdunsten den Wirkstoff im Depot zurücklässt. Als Lösemittel können beispielsweise Pentan, Hexan, Heptan, Aceton, Ethanol, Methanol oder andere leicht flüchtige organische Lösungsmittel verwendet werden. Nach dem Verdunsten des Lösungsmittels ist der Wirkstoff wie gewünscht an der inneren Oberfläche der nanoporösen Partikel adsorptiv gebunden, ohne den Luftzutritt entscheidend zu behindern. Der Wirkstoff liegt zusätzlich auch an der äußeren Oberfläche der Partikel vor, wobei die innere, von den Nanoporen bereitgestellte Oberfläche größer als die äußere Oberfläche der Partikel und des Stützgerüstes ist.

Nanoporöse Materialien mit einer geeigneten Affinität (der Porenoberfläche) zum Wirkstoff (Aromastoff) und die Zugabe einer Wirkstoffmenge (Menge des Aromastoffs) entsprechend der Beladungs- bzw. Adsorptionskapazität des nanoporösen Materials erlauben somit einerseits eine hohe Beladung des Depots mit Wirk- und oder Aromastoffen und andererseits deren schnelle Freisetzung in ein vorbei- oder durchströmende Gas oder Gasgemisch. Dabei kann der Gastransport im Inneren der porösen Partikel überwiegend durch Diffusion erfolgen.

Die Beladung des Depots mit Wirk- und/oder Aromastoffen kann vorteilhaft nach dem Sinterschritt erfolgen. Ebenso ist es jedoch bei stabilen Wirkstoffen möglich, die Beladung der nanoporösen Partikeln während oder vor dem Sinterschritt vorzunehmen.

Ein besonderer Vorteil der an der Oberfläche eines makroporösen Stützgerüsts dauerhaft durch Sintern befestigten nanoporösen Partikeln mit hoher spezifischer Oberfläche und Affinität für Wirk- und Aromastoffe und deren Verwendung in einem Depot zur Substanz-Freisetzung in durchströmende Luft besteht darin, dass lediglich die jeweils zuvor gebundenen Substanzen freigesetzt werden. Im Luftstrom, welcher das Depot verlässt, treten neben den gasförmig vorliegenden Wirk- und/oder Aromastoffen keine Flüssigkeitströpfchen irgendwelcher Hilfssubstanzen oder gar gesundheitsgefährdende Partikel auf. Die Fixierung der nanoporösen Partikel am makroporösen Stützgerüst verhindert, dass die nanoporösen Partikel vom Gasstrom mitgerissen werden können.

Um die Affinität der nanoporösen Materialien sowohl gezielt in Richtung einer optimalen Aufnahme als auch Freisetzung zu modifizieren, wurden verschiedene Möglichkeiten der Vorbehandlung der Porenoberflächen am Beispiel einer vorausgehenden Reinigung, einer Aktivierung, einem Anätzen mit Säuren oder Basen sowie einer Silanisierung untersucht.

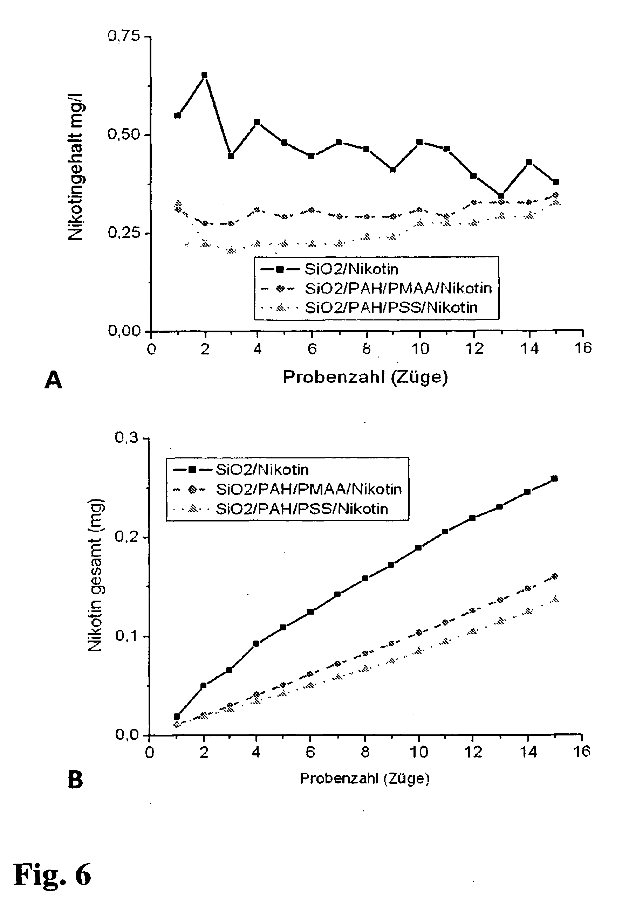

Überraschenderweise erwies sich eine einfache Beschichtung der Poren mit Polyelektrolyten nach der Layer-by-Layer- (LbL-)Technik als besonders geeignet, um die Aufnahme bzw. Freisetzung von Wirkstoffen zu steuern. Die LbL-Beschichtung von Nanoporen zu deren Funktionalisierung ist in der Patentanmeldung DE 10 2004 013 637 offenbart. Beispielsweise ist es möglich, durch eine derartige Modifikation mit Polyelektrolyt-Schichten die Freisetzung von Wirkstoffen zu verzögern (Vergleiche dazu Figur 6 sowie Beispiel 7).

Eine oder mehrere Ausführungsformen nutzen die Temperaturabhängigkeit des Adsorptionsgleichgewichtes der Wirkstoffe bzw. der Aromastoffe an nanoporösen Partikeln. So kann durch die eingestellte Temperatur des anströmenden Gases oder Gasgemischs, zum Beispiel Luft, die jeweils in das durchströmende Volumen freigesetzte Menge der Wirkstoffe und/oder Aromastoffe eingestellt werden. Diese Einstellung der jeweils freigesetzten Substanzmenge kann in Abhängigkeit von den Erfordernissen der jeweiligen Anwendung erfolgen. Neben der Einstellung der Affinität der Oberfläche der nanoporösen Partikel für die Wirkstoffe bzw. Aromastoffe kann deren Freisetzung somit auch über die Temperatur des das Depot durchströmenden Gases oder Gasgemischs geregelt werden. Nach einer oder mehreren Ausführungsformen kann die Freisetzung auch über die Temperatur des Depots selbst geregelt werden.

Beispielsweise ist es möglich, die beim Ablauf einer exothermen Reaktion freigesetzte Wärme zur zusätzlichen Modulation der Freisetzungskinetik des Wirkstoffs zu nutzen. Ebenso kann ein elektronisch gesteuertes Heizelement zur Aufheizung des Gases vor dem Durchströmen des Depots oder zur Aufheizung des Depots selbst für eine Regulierung der freigesetzten Wirkstoffmenge eingesetzt werden.

Nach einer oder mehreren Ausführungsformen kann das Depot in Kombination mit einer elektrischen, optischen, chemischen, physikalischen oder natürlichen Heizquelle zum Vorwärmen des Gases oder zur Erwärmung des Depots genutzt werden, um die Menge der abgegebenen Substanz zu erhöhen bzw. kontrolliert zu steuern. Beispielsweise kann eine rauchfreie Zigarette eine elektrische Heizquelle aufweisen.

Die Verfahrensschritte zur Herstellung eines hier beschriebenen Depots für Wirkstoffe und/oder Aromastoffe umfassen gemäß einer oder mehrerer Ausführungsformen die Auswahl einer mikropanikulären Matrix zur Einbettung nanoporösen partikulären Materials, die Auswahl eines nanoporösen partikulären Materials, die gezielte Einstellung der Affinität des nanopartikulären Materials durch Modifikation, die Kosinterung des nanoporösen Materials mit dem Stützmaterial bei einer Temperatur nahe der Schmelz- oder Erweichungstemperatur des als Stützmatrix verwendeten Materials, sowie die Adsorption von Wirk- und/oder Aromastoffen an der Oberfläche der Nanoporen des nanoporösen Materials. Dabei kann eine gezielte Einstellung der adsorbierten Wirkstoffmenge erfolgen.

Als Matrix für die Fixierung nanoporöser Partikel können anorganische Materialien, wie Glas, Silikate oder Alumosilikate oder thermoplastische organische Polymere verwendet werden.

Nach einer oder mehreren Ausführungsformen kann bei der Auswahl des nanoporösen partikulären Materials auch auf den Schritt der gezielten Einstellung der Affinität für die Wirkstoffe oder Aromastoffe verzichtet werden. Wie oben erläutert, können die Verfahrensschritte Kosinterung und Adsorption bzw. Beladung auch miteinander kombiniert werden.

Nanoporöse Materialien aus Siliziumdioxid oder Alumosilikaten werden heutzutage in sehr verschiedenen Ausführungen hergestellt.

Gemäß einer oder mehreren Ausführungsformen wird ein Verfahren zur Herstellung eines Depots bereitgestellt. Das Verfahren weist auf das Herstellen einer Hybridstruktur, umfassend ein offenporiges makroporöses Stützgerüst, durch das ein Gas strömen kann, und am Stützgerüsts immobilisierte, nanoporöse Partikel, die in ihren Poren wenigstens einen freizusetzenden Wirkstoff enthalten, wobei das Stützgerüst aus dauerhaft miteinander verbundenen Partikeln oder Fasern besteht.

Gemäß einer oder mehreren Ausführungsformen wird ein Verfahren zur Herstellung eines Depots bereitgestellt. Das Verfahren weist auf das Herstellen einer Hybridstruktur, umfassend ein offenporiges makroporöses Stützgerüst, durch das ein Gas strömen kann, und im Material des Stützgerüstes ausgebildete Nanoporen, wobei das Stützgerüst im Wesentlichen aus einem anorganischen Material, wie Glas, Silikaten oder Alumosilikaten besteht, und wobei die Poren wenigstens einen freizusetzenden Wirkstoff enthalten.

Gemäß einer oder mehreren Ausführungsformen wird das offenporige Stützgerüst, durch Sintern von Mikropartikeln hergestellt. Gemäß einer oder mehreren Ausführungsformen werden die nanoporösen Partikel zusammen mit den Mikropartikeln gesintert. Gemäß einer oder mehreren Ausführungsformen werden die Nanoporen durch Auslaugen von Salzen aus dem Material des Stützgerüsts gebildet. Gemäß einer oder mehreren Ausführungsformen wird der zumindest eine Wirk- oder Aromastoff nach Bildung der Nanoporen bzw. Immobilisierung der nanopörösen Partikel in den Poren adsorbiert.

Folgende Kriterien können bei der Auswahl für das beschriebene Depot genutzt werden.

### 1. Beladungskapazität

Für ein möglichst geringes Depotvolumen, ist eine hohe Beladbarkeit der nanoporösen Materialien erwünscht. Die Beladungskapazität wird hier prozentual als Masse Wirkstoff pro Masse des gefüllten Partikels angegeben. Geeignete Beladungskapazitäten liegen zwischen 20 % und 90%, beispielsweise zwischen 30% bis 85%, insbesondere zwischen 60% und 80%.

### 2. Freisetzbarkeit

In Materialien mit sehr hoher Affinität zum Wirkstoff ist der Verteilungskoeffizient zwischen Luft und Oberfläche sehr zu letzterer hin verschoben. Demzufolge ist die Gleichgewichtskonzentration des Wirkstoffs in der Luft gering. Insofern sollte die Affinität zu den Wirkstoffen gerade ausreichend sein, um eine effektive Adsorption zu gewährleisten. Für das Einstellen der Wechselwirkungen zwischen Porenoberfläche und Wirkstoff, die die Affinität bestimmen, wird eine Vorbeschichtung der nanoporösen Partikel mit Polyelektrolyten mit Hilfe der LbL-Technik vorgeschlagen. Je nach Wirkstoff kann der komplementäre Polyelektrolyt so gewählt werden, dass eine kationische, anionische, wasserstoffbrückenbildende oder hydrophobe Oberfläche präsentiert wird.

### 3. Größe der nanoporösen Partikel

Dieser Parameter bestimmt wesentlich das Design des Depotfilters. Ist die Größe der nanoporösen Partikel zu gering, können sie bei ungenügender Fixierung und Filterung mitgerissen werden und aus dem Depot entweichen und beispielsweise in den Atemtrakt des Inhalierenden gelangen. Sind die Partikel zu groß, ist der Austausch der Luft mit dem Wirkstoff, z.B. Nikotin, im Inneren der Partikel unzureichend und die Makroporen des Gerüstes sind verstopft. Geeignet sind beispielsweise Größenverteilungen für die nanoporösen Partikel von 10 µm bis 1 mm, insbesondere von 50 µm bis 700 µm. Besonders geeignet sind irreguläre Partikel einer engen Größenverteilung.

### 4. Porenweite

Die Porenweite der nanoporösen Partikel bestimmt einerseits die verfügbare Oberfläche und somit die Menge des adsorbierten Wirkstoffs bzw. Aromastoffs und andererseits die Geschwindigkeit des Luftaustausches. Während abnehmende Porenweiten zu erhöhter Beladungskapazität, beispielsweise für Nikotin führen, nimmt gleichzeitig der diffusive Luftaustausch ab. Für die Verwendung in einem Depot erwies es sich als vorteilhaft, Materialien mit keiner zu engen Porenverteilung zu verwenden. Porendurchmesser mit einer breiten Verteilung von 1 nm bis 900 nm, insbesondere im Bereich von 3 nm bis 700 nm, sind für eine effektive Adsorption geeignet. Dabei sollte die innere Oberfläche, gemessen durch die BET Stickstoffadsoiption, beispielsweise 30-1000 m²/g, vorteilhafterweise 100-600 m²/g betragen.

### 5. Fixierung der nanoporösen Partikel in der makroporösen Matrix

Um ein gleichmäßiges Anströmen der den Wirkstoff enthaltenden nanoporösen Partikel mit dem durchgeleiteten Gas oder Gasgemisch, beispielsweise Luft, zu ermöglichen, den Austritt der Partikeln in ggf. eingeatmete Luft jedoch zu verhindern, sollten sie im Depot stabil fixiert werden. Eine vorteilhafte Ausführungsform eines Depots stellt die Kosinterung der erst bei höheren Temperaturen schmelzenden nanoporösen Partikel mit größeren Sintergranulaten aus Polymeren dar, die einen niedrigeren Schmelz- bzw. Erweichungspunkt besitzen. Der Anteil nanoporöser Partikeln im Sinterstück kann insbesondere zwischen 2% und 40%, beispielsweise zwischen 5% bis 20% liegen. Der Anteil der nanoporösen Partikel sollte dabei so gewählt werden, dass das gesinterte oder angeschmolzene Stützgerüsts noch ausreichend mechanisch stabil ist. Beispielsweise können Anteile nanoporöser Partikeln höher als 40% in einigen Fällen nicht mehr die Integrität des makroporösen Stützgerüsts gewährleisten.

Mit einem Modellexperiment konnte gezeigt werden, dass die inneren Poren immobilisierter Partikeln für kleinere Moleküle, inbesondere für Luft, zugänglich sind.

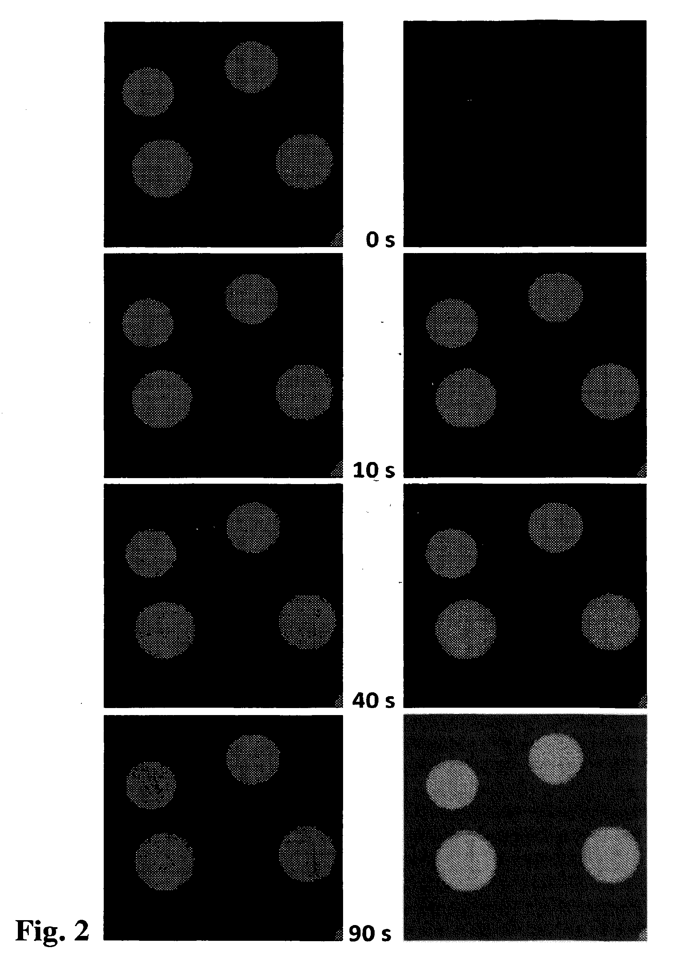

Nanoporöse Partikeln wurden mit Cy5 gelabeltem Polymer inkubiert. Dabei dringt das Polymer vollständig in die Poren ein und wird an deren Oberfläche adsorbiert. Mittels konfokaler Mikroskopie wurde dann gezeigt, dass der niedermolekulare Fluoreszenzfarbstoff Sulforhodamin innerhalb kurzer Zeit in diese Poren eindringt, ohne den anderen Farbstoff zu verdrängen (Vergleiche Figur 2).

Die linke Spalte in Figur 2 zeigt die im Cy5 Kanal eines konfokalen Fluoreszenzmikroskops beim Zoomfaktor 4 und einer Verstärkerspannung des Fotomultiplier von 570 V aufgenommenen Schnittbilder der nanoporösen Partikel (Durchmesser 10 µm) mit dem fest an der inneren Oberfläche der Poren adsorbierten Polymer, während die rechte Spalte die Bilder des Rhodamin-Kanals, bei Zoomfaktor 4 und einer Spannung des Fotomultiplier von 740 V zeigt. Die angegebene Inkubationszeit (Sekunden) zeigt das Eindringen einer 10⁵ M Lösung des Fluoreszenzfarbstoffes Sulforhodamin in die Poren der Partikel. Bereits nach 10 s waren die Partikel bis ins Innere komplett mit dem Sulforhodamin befüllt, wobei radial nahezu kein Gradient zu beobachten ist. Das heißt, auch das Innere der Partikel ist ohne große Diffusionshemmung zugänglich. Die Original-Bildgröße beträgt 40 µm x 40 µm.

Das bedeutet, dass im Gegensatz zu einer durch Kapillarwirkungen in Mikroporen aufgesaugten Flüssigkeit, die Nanoporen durch eine adsorbierte Schicht nicht verschlossen werden. Auch Gasmoleküle, z.B. der Luft, können durch Diffusion oder auch Konvektion so problemlos ins Innere und wieder heraus gelangen, was für einen schnellen Austausch der Wirkstoffe wesentlich ist.

Gemäß einer oder mehrerer Ausführungsformen zur Kombination von Makro- mit Nanoporosität werden Silikabeads im Größenbereich von 50 µm bis 1000 µm, beispielsweise von 100 µm bis 700 µm zusammengesintert und in nachfolgenden Schritten durch Auslaugen von Salzen nanoporös gemacht. Dafür geeignete Techniken sind in der Fachliteratur dargelegt.

Eine oder mehrere Ausführungsformen des Depots stützen sich auf die Freisetzung von Wirkstoffen oder Aromastoffen aus deren Salzen. So kann zum Beispiel in einem ersten Depot Ammoniumcarbonat immobilisiert vorliegen, das beim Durchströmen von Luft Ammoniak freisetzt. Liegt in einem nachgeschalteten weiteren Depot ein Salz des Wirkstoffs, beispielsweise Nikotinacetat vor, so wird bei Reaktion mit Ammoniak die freie, flüchtige Nikotinbase freigesetzt. Ein Ammoniak freisetzendes Depot kann auch in einer anderen Ausführungsform als Vorfilter für ein Hybriddepot des freien Nikotins dienen, um die bei Inhalation der freien Nikotinbase auftretende Reizung des Mund- und Rachenraumes durch den Ammoniak abzumildern. Dieser Effekt wird bei normalen Zigaretten ebenfalls genutzt.

Gemäß einer oder mehrerer Ausführungsformen können mindestens zwei Depots hintereinander angeordnet werden, die mit unterschiedlichen Substanzen beladen sind. Die hintereinander angeordneten Depots können beispielsweise in einer Kartusche "hintereinandergeschaltet" sein.

Anhand der beigefügten Zeichnungen werden nun Ausführungsbeispiele erläutert.

1. Beispiel: Jeweils 20 mg nanoporöse sphärische chromatographische Trägerpartikel mit einer inneren (BET) Oberfläche von 600 m²/g und mit einem Durchmesser von 10 µm und einer engen Porenweitenverteilung eines Porendurchmessers von 12 nm wurden mit 80 mg Polyethylenbeads eines Durchmessers von 500 µm fein vermischt, in eine aus einem Glasrohr gefertigte Form überführt und 15 min bei 118°C gesintert. Das ergab ein Hybridfilterdepot mit einem Durchmesser von 6 mm und einer Dicke von 9 mm. Das Depot wurde mit 6 mg Nikotin beladen, indem das Nikotin in 0,5 mL Ethanol aufgelöst wurde und auf das in einem kleinen zylindrischen Gefäß befindliche Depot gegeben wurde. Nach vollständigem Verdunsten des Ethanols wurden 35 mL auf 60°C erwärmte Luft durch das Depot in 1 bis 2 Sekunden gesaugt. In der angesaugten Luft wurde dann der Gehalt an Nikotin bestimmt. Das erfolgte durch Ausschütteln mit 0,1 M Salzsäure und anschließender spektroskopischer Analyse (ε_{259nm,pH1} = 4040 l/mol x cm).

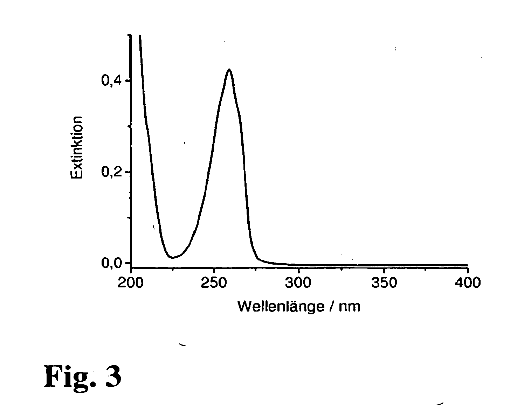

Figur 3 zeigt das UV/Vis Absorptionsspektrum von 0,107 mM Nikotin in 0,1 M Salzsäure. Der Nikotingehalt der durch das Depot gesaugten Luft beträgt bei den ersten 15 Zügen weitgehend konstant 1,15 mg/L.

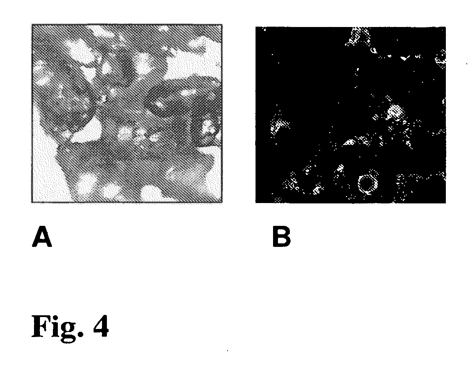

2. Beispiel: Nanoporöse sphärische Partikel eines Magnesiumalumometasilikates mit einem Durchmesser von 50 µm bis 150 µm und einer Porenweitenverteilung zwischen 5 nm bis 300 nm wurden mit Polyethylenbeads zu einem makroporösen Depot mit einem Durchmesser von 5,7 mm und einer Dicke von 3 mm gesintert. Die Fixierung erfolgte durch Co-Sinterung mit Polyethylenbeads einer Größe von 500 µm für 15 min bei 118°C. Die mikroskopische Aufnahme des erhaltenen stabilen Konglomerats zeigt das makroporöse Depot mit den eingebetteten nanoporösen Partikeln (Vergleiche Figur 4).

Figur 4 zeigt Mikroskopaufnahmen des gesinterten Hybridfilterdepots. A-Durchlicht-Modus. Gut zu erkennen sind die etwa 200 µm großen Kanäle in dem makroporösen Gerüst. B - konfokale Fluoreszenzaufnahme der eingebetteten Alumosilikatpartikel, die mit Rhodamin 6G selektiv angefärbt sind (Bildgröße 1 mm x 1 mm).

Es wurde ein Masseverhältnis von 31,5 mg Polyethylen und 3,2 mg nanoporösen Partikeln gewählt, was für eine anschließende Befüllung mit 5 mg Nikotin ausreichte. Dabei lagerte sich das Nikotin überwiegend in den nanoporösen Partikeln ab. Das Depot war nach der Befüllung weitgehend farblos. Durch dieses Depot wurden 35 mL 60°C warme Luft in der Zeit eines üblichen Raucherzuges gesaugt. Der Nikotingehalt in der durch das Depot angesaugten Luft betrug 1,4 mg/L.

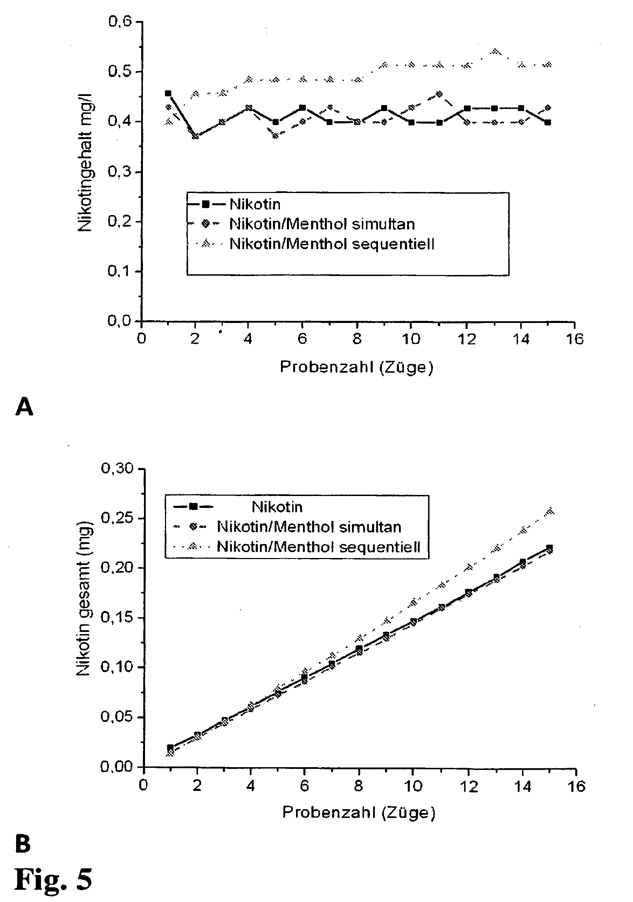

3. Beispiel: Nanoporöse sphärische Partikel einer Fällungskieselsäure mit einem mittleren Durchmesser von 100 µm und einer inneren (BET) Oberfläche von 150 - 300 m²/g und einer besonders breiten Porenweitenverteilung zwischen 3 nm und 700 nm wurden zur Herstellung eines Depotfilters verwendet. Die Fixierung erfolgte wie im zweiten Beispiel beschrieben. Es wurden 5,2 mg der nanoporösen Partikel mit 31,5 mg Polyethylen-Kügelchen gesintert, was für eine Befüllung mit 10 mg Nikotin ausreichte. Durch dieses Depot wurden 35 mL Luft mit Raumtemperatur in der Zeit eines üblichen Raucherzuges gesaugt und der Nikotingehalt in der angesaugten Luft bei 15 aufeinander folgenden Zügen bestimmt (Vergleiche Figur 5).

Figur 5 zeigt die gemessene Nikotinfreisetzung pro Inhalationszug (35 mL) mit Raumluft. A - Einzelmessungen, Nikotingehalt in angesaugter Luft; B - akkumulierte Werte der Freisetzung von Nikotin. Die durchgehende Linie (quadratische Datenpunkte) zeigt den entsprechenden Kurvenverlauf für eine Nikotinbefüllung mit 10 mg.

Es wurden innerhalb der Zugzahl einer normalen Zigarette weitgehend gleiche Nikotin-Mengen in die angesaugte Luft freigesetzt, was auf eine sehr zuverlässige und über die Menge der eingesinterten Partikel auch gut einstellbare Nikotinabgabe hinweist. Die Untersuchung wurde an 3 parallel hergestellten Depots durchgeführt und ergab bei allen Zügen nahezu identische Werte von etwa 0,4 mg/L. Nach DIN ISO 3308: 2000-12 entspricht das mit 15 Zügen je 35 mL beim Rauchen einer Zigarette einer Gesamtmenge von 0,22 mg Nikotin. Dieser Wert entspricht der inhalierten Nikotinmenge einer sehr leichten Zigarette

4. Beispiel: Ein Depotfilter nach Beispiel 3 wurde zusätzlich zur Befüllung mit 10 mg Nikotin mit 1 mg Menthol befüllt. Die durch dieses Depot eingeatmete Luft hatte einen starken Mentholgeschmack, der z.B. einer Mentholzigarette vergleichbar ist. Durch dieses Depot wurden 15 mal 35 mL Luft mit Raumtemperatur in der Zeit eines üblichen Raucherzuges gesaugt und eine gleiche Nikotinfreisetzung wie im Falle des dritten Ausführungsbeispiels gemessen (Vergleiche Figur 5). Die gestrichelte Linie (kreisförmige Datenpunkte) zeigt den Kurvenverlauf für die simultane Befüllung mit 10 mg Nikotin und 1 mg Menthol in einem Depot.

5. Beispiel: Es wurde ein Doppelfiltersystem hergestellt mit 1 mg Menthol in einem Depotfilter nach Beispiel 3 und 10 mg Nikotin in einem weiteren, nachgeschalteten Depot nach Beispiel 3. Die durch diese Depotkombination eingeatmete Luft hatte einen starken Mentholgeschmack, der z.B. einer Mentholzigarette vergleichbar ist. Durch dieses Doppeldepot wurden 15 mal 35 mL Luft mit Raumtemperatur in der Zeit eines üblichen Raucherzuges gesaugt und eine etwas höhere Nikotinfreisetzung als im Falle des 3. und 4. Ausführungsbeispiels gemessen (Vergleiche Figur 5). Die gepunktete Linie (dreieckige Datenpunkte) zeigt die Kurvenverläufe für das Doppel-Depotsystem. Offensichtlich können durch synergistische Effekte zwischen verschiedenen, freigesetzten Substanzen auch höhere Freisetzungen als bei der Freisetzung einzelner Substanzen erreicht werden.

6. Beispiel: Die nanoporösen Partikel in einem Depot nach Beispiel 3 wurden vor der Befüllung mit Polyallylamin(PAH)/Polystyrolsulfonat(PSS) bzw. Polyallylamin(PAH)/Polymethacrylat(PMAA) beschichtet, um die negative Ladung an der Oberfläche und damit die Affinität zum Nikotin zu erhöhen bzw. die Freisetzungsrate zu reduzieren. Nach Befüllung mit 10 mg Nikotin wurde die Freisetzung bei Raumtemperatur getestet, die bei den beschichteten Partikeln bei gleicher Befüllmenge wesentlich niedriger lag (Vergleiche Fig. 6).

Figur 6 zeigt die gemessene Nikotinfreisetzung pro Inhalationszug von 35 mL Raumluft. Fig. 6A zeigt Einzelmessungen, Fig. 6B zeigt die akkumulierte Freisetzung. Dabei zeigt die durchgezogene Linie (quadratische Datenpunkte) die gemessene Nikotinfreisetzung bei einer Nikotinbefüllung mit 10 mg durch unmodifizierte nanoporöse Partikel. Die gestrichelte Linie (kreisförmige Datenpunkte) zeigt die gemessene Nikotinfreisetzung bei einer Befüllung mit 10 mg Nikotin durch Partikel deren Poren mit Poly(allylamin) (PAH) und danach mit Poly(methacrylsäure) (PMAA) beschichtet wurden. Die gepunktete Linie (dreieckige Datenpunkte) stellt den Verlauf der gemessenen Nikotinfreisetzung nach einer Erhöhung der Affinität des nanoporösen Partikels zu Nikotin durch die in Beispiel 6 beschriebene LbL Beschichtung mit Poly(allylamin)/Poly(styrolsulfonat) bei 10 mg Nikotinbefüllung dar.

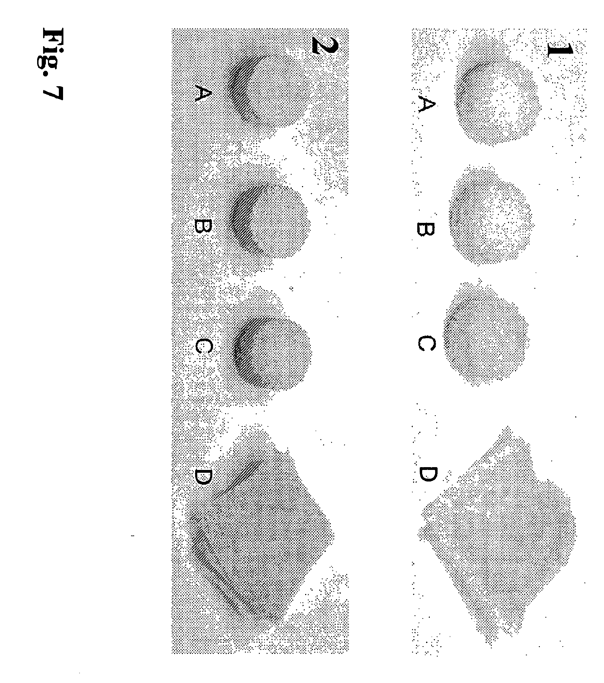

7. Beispiel: Stabilitätstestung: Es wurden Depots nach Beispiel 3 hergestellt und mit verschiedenen Mengen Nikotin befüllt (A: 8 mg, B: 10 mg und C: 12 mg). Zum Vergleich wurden 10 mg Nikotin auf ein-Acetatfaserpapier aufgebracht (D). Die obere Reihe in Figur 7 (Bild 1) zeigt die unmittelbar nach Nikotinbeladung aufgenommenen Proben.

Diese Proben wurden anschließend in einem geschlossenen Gefäß 48 h bei 60°C gelagert. Die untere Reihe in Figur 7 (Bild 2) zeigt die Depots nach Lagerung unter den angegebenen Stress-Bedingungen, was vergleichbar mit einer mehirmonatigen Lagerung bei Raumtemperatur ist. Wie klar zu erkennen ist, ist das Nikotin auf dem Acetatpapier deutlich braun geworden. Die Depots A und B hatten sich nur geringfügig durch die Zersetzungsprodukte gefärbt, wohingegen Depot C ebenfalls eine starke Gelb/Braunfärbung zeigt. Das liegt daran, dass die Aufnahmekapazität des im Depot befindlichen nanoporösen Materials bei einer Menge von 10 mg Nikotin erschöpft ist und somit freies Nikotin auch außerhalb der Partikel in dem makroporösen Polyethylen-Gerüst ablagert wurde.

8. Beispiel: Es wurden 2 Depots nach Beispiel 3 hergestellt. Eines der Depots wurde mit 10 mg Eukalyptusöl in Methanol, das andere mit 10 mg Latschenkieferöl in Cyclohexan befüllt. Die Depots wurden jeweils in einem Trägerrohr positioniert und dann mit moderater Geschwindigkeit Luft mit Raumtemperatur hindurchgeleitet. Die abgeleitete Luft wurde in einen 20 L Kanister eingeleitet. Trotz des großen Luftvolumens war in beiden Fällen ein intensiver Duft nach Eukalyptus bzw. Latschenkieferöl nachzuweisen.

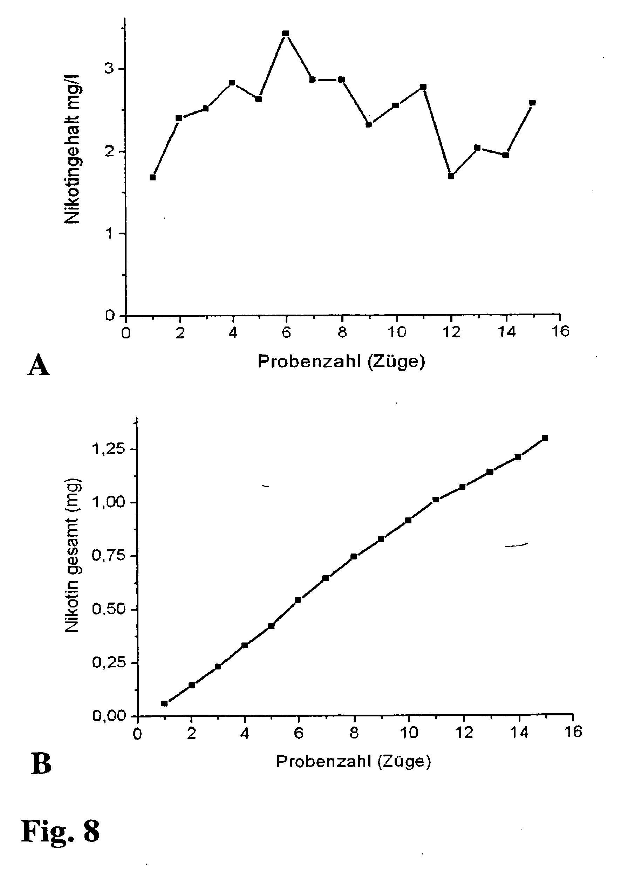

9. Beispiel: Nanoporöse sphärische Partikel einer Fällungskieselsäure mit einem Durchmesser von 50 µm und einer inneren (BET) Oberfläche von 150 - 300 m²/g und einer Porenweitenverteilung zwischen 3 nm und 700 nm und einer mittleren Adsorptionskapazität wurden zur Herstellung eines Depotfilters verwendet. Die Fixierung erfolgte wie im 2. Beispiel beschrieben. Es wurden 5,2 mg der nanoporösen Partikel mit 31,5 mg Polyethylenbeads gesintert, was für eine Befüllung mit 10 mg Nikotin ausreichte. Durch dieses Depot wurden 15 x 35 mL Luft in der Zeit eines üblichen Raucherzuges gesaugt. Die Luft wurde mit einem vorgeschalteten Heizstab auf etwa 60-80°C erwärmt und der Nikotingehalt in der angesaugten Luft bestimmt (Vergleiche Figur 8A). Die Freisetzung erfolgte hier sehr ungleichmäßig, da die Temperatur stark schwankte. Der Gesamtnikotingehalt nach 15 Zügen (Vergleiche Figur 8B) entsprach 1,3 mg, was dem Nikotingehalt einer sehr starken Zigarette entspricht.

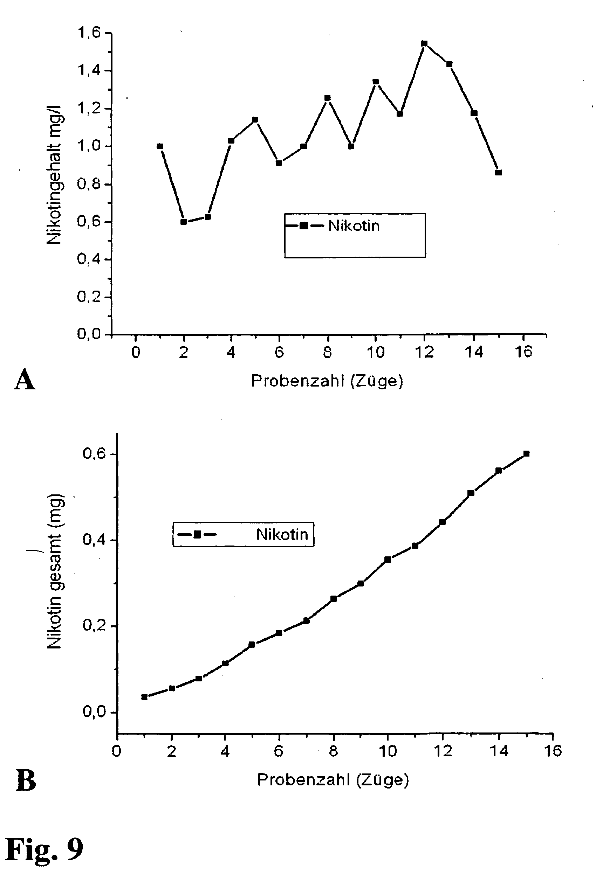

10. Beispiel: Glasbeads einer Größe von 300 µm bis 500 µm aus einem Alkaliborosilikatglas wurden zu einem makroporösen Gerüstzylinder eines Durchmessers von 6 mm und einer Höhe von 8 mm zusammengesintert. Nach einer Phasenseparation im Glas bei Temperaturen zwischen 400°C und 600°C wurde die lösliche Boratphase im Sauren herausgelaugt und damit eine Nanoporosität in den Beads erreicht. Mit einer anschließenden basischen Laugung wurden die Poren für Wirkstoffe bzw. Luft durchlässig gemacht. Das erhaltene Hybriddepot wurde mit 10 mg Nikotin befüllt und dessen Freisetzung in durchgesaugte Luft einer Temperatur von 60°C mit 15 Zügen zu jeweils 35 mL bestimmt. Die Luft wurde hier in einem Wasserbad vorgewärmt und hatte daher eine weitgehend konstante Temperatur. Innerhalb der 15 Züge wurde eine leicht zunehemende Konzentration an Nikotin von 0.8 - 1, 3 mg/l gemessen. (Vergleiche Fig. 9)

Die vorliegende Erfindung wurde anhand von Ausführungsbeispielen erläutert. Diese Ausführungsbeispiele sollten keinesfalls als einschränkend für die vorliegende Erfindung verstanden werden. Die nachfolgenden Ansprüche stellen einen ersten, nicht bindenden Versuch dar, die Erfindung mit allgemeinen Worten zu beschreiben.

### REFERENZEN

WO 2004/098324
WO 2004/095955
CN 101268867A (WO 2009/105919)
DE 10 2008 014 587
DE 10 2005 054 255 A1
DE 103 56 925 B4
WO 2006/002 445 A2
AT 505 472 A1
DE 10 2004 013 637
C. Peyratout, L. Dähne (2004) "Tailor-made Polyelectrolyte Microcapsules: From Multilayers to Smart Containers." Review in Angew. Chem. Int. Ed. 43, 3762 - 3783.
F. Janowski, D. Enke (2002) "Porous Glasses." In: Handbook of Porous Solids, (F. Schüth, K.S.W. Sing, J. Weitkamp - Eds.) Band 3, Wiley-VCH, Weinheim 2002, 1432.
D. Enke, F. Janowski, W. Schwieger (2003) "Porous glases in the 21st century-a short review." Microporous and Mesoporous Materials, 60, 19-30.
DIN ISO 3308: 2000-12 (Zigaretten-Abrauchmaschine für Routineanalysen - Begriffe und Standardbedingungen)
DIN ISO 9277:2003-05 (Bestimmung der spezifischen Oberfläche von Feststoffen durch Gasadsorption nach dem BET-Verfahren)

### BEZUGSZEICHENLISTE

1 - Partikel des Stützgerüsts
2 - Poren und Kavernen im Stützgerüst
3 - Nanoporöses Partikels
4 - Schematische Schnittebene durch das Stützgerüst
5 - Poren des nanoporösen Partikels
6 - Adsorbierter Wirkstoff

## Patentansprüche

1. Depot für die Speicherung und Abgabe einer Substanz in eine Gasphase, aufweisend eine Hybridstruktur aus
- einem offenporigen makroporösen Stützgerüst, durch das ein Gas strömen kann, und
- am Stützgerüst immobilisierten, nanoporösen Partikeln, die in ihren Nanoporen wenigstens eine freizusetzende Substanz enthalten,
wobei das Stützgerüst aus dauerhaft miteinander verbundenen Partikeln oder Fasern besteht.

2. Depot nach Anspruch 1, wobei die nanoporösen Partikel aus einem anderen Material als das Stützgerüst bestehen.

3. Depot für die Speicherung und Abgabe einer Substanz in eine Gasphase, aufweisend eine Hybridstruktur aus
- einem offenporigen makroporösen Stützgerüst, durch das ein Gas strömen kann und
- im Material des Stützgerüstes ausgebildeten Nanoporen,
wobei das Stützgerüst im Wesentlichen aus einem anorganischen Material, wie Glas, Silikate oder Alumosilikate besteht, und wobei die Nanoporen wenigstens eine freizusetzende Substanz enthalten.

4. Depot nach einem der Ansprüche 1 bis 3, wobei
die Substanz ein pharmazeutischer Wirkstoff oder ein Aromastoff ist,
der in den Nanoporen adsorbiert oder gebunden vorliegt.

5. Depot nach einem der Ansprüche 1 bis 3, wobei
die Substanz Nikotin, ein Nikotinsalz oder ein Inhaltsstoff von Zigarren- oder Zigarettentabak ist, der in den Nanoporen adsorbiert oder gebunden vorliegt.

6. Depot nach einem der Ansprüche 1 bis 3, wobei die Substanz
ein Insektizid, ein Akarizid oder ein Rodentizid ist, oder
ein biologischer Wirkstoff ist, wie Cinerin, Jasmolin, oder Pyrethrin, oder
ein Repellent für die Vergrämung von Haus- oder Wildtieren ist, oder
ein Insekten-Repellent ist, wie p-Menthan-diol, Diethyltoluamid oder Permethrin und in den Nanoporen adsorbiert oder gebunden vorliegt.

7. Depot nach einem der Ansprüche 1 bis 3,wobei
die Substanz ein Pheromon zum gezielten Anlocken von Insekten ist.

8. Kartusche für eine Dosiervorrichtung, umfassend:
- einen Zylinder, wobei der Zylinder zumindest eine Einlass-Öffnung und zumindest eine Auslass-Öffnung hat,
- ein Depot nach einem der Ansprüche 1 - 7, wobei das Depot in dem Zylinder zwischen der Einlass- und der Auslass-Öffnung gehalten wird.

9. Rauchfreie Zigarette, Zigarillo, Zigarre oder Tabakspfeife, aufweisend einen Grundkörper und wenigstens ein im Grundkörper angeordnetes Depot nach einem der Ansprüche 1 bis 3, wobei die Substanz Nikotin, ein Nikotinsalz, Nikotinderivat oder ein Inhaltsstoff von Zigarren- oder Zigarettentabak ist, die in den Nanoporen adsorbiert oder gebunden vorliegt.

10. Rauchfreie Zigarette, Zigarillo, Zigarre oder Tabakspfeife nach Anspruch 9, wobei die Zigarette, Zigarillo, Zigarre oder Tabakspfeife eine Heizquelle zum Vorwärmen des Gases oder zur Erwärmung des Depots aufweist.

11. Vorrichtung, adaptiert zur Freisetzung einer Substanz in einen Gasstrom, aufweisend ein Depot nach einem der Ansprüche 1 bis 7 oder eine Kartusche nach Anspruch 8, wobei der Gasstrom durch das Depot leitbar ist.

12. Verwendung eines Depots nach einem der Ansprüche 1 bis 7 oder einer Kartusche nach Anspruch 8 zur Abgabe einer Substanz in ein Gasvolumen.

13. Verwendung eines Depots oder Kartusche nach Anspruch 12 in Kombination mit einer Heizquelle zum Vorwärmen des Gases oder zur Erwärmung des Depots, um die Menge der abgegebenen Substanz zu erhöhen bzw. kontrolliert zu steuern.

## Claims

1. A depot for storing and releasing a substance into a gas phase, comprising a hybrid structure formed by:
- an open-pored macroporous support matrix through which a gas can flow; and
- nanoporous particles immobilized on the support matrix, the nanopores of the nanoporous particles containing at least one substance which is to be released;
wherein the support matrix is comprised of permanently interconnected particles or fibres.

2. The depot as claimed in claim 1, wherein the nanoporous particles comprised of a material other than that of the support matrix.

3. A depot for storing and releasing a substance into a gas phase, comprising a hybrid structure formed by:
- an open-pored macroporous support matrix through which a gas can flow; and
- nanopores formed in the material of the support matrix;
wherein the support matrix essentially consists of an inorganic material such as glass, silicates or aluminosilicates, and wherein the nanopores contain at least one substance to be released.

4. The depot as claimed in one of claims 1 to 3, wherein the substance is a pharmaceutically active substance or a flavouring agent which is adsorbed onto or bound into the nanopores.

5. The depot as claimed in one of claims 1 to 3, wherein the substance is nicotine, a nicotine salt or an ingredient of cigars or cigarette tobacco, which is adsorbed onto or bound into the nanopores.

6. The depot as claimed in one of claims 1 to 3, wherein the substance is
an insecticide, an acaricide or a rodenticide, or
a biologically active substance such as cinerin, jasmolin or pyrethrin, or
a repellent to repel pets or wild animals or
an insect repellent such as p-menthane diol, diethyltoluamide or permethrin
and is adsorbed onto or bound into the nanopores.

7. The depot as claimed in one of claims 1 to 3, wherein the substance is a pheromone to deliberately attract insects.

8. A cartridge for a dosing device, comprising:
- a cylinder, wherein the cylinder has at least one inlet opening and at least one outlet opening;
- a depot as claimed in one of claims 1 - 7, wherein the depot is held in the cylinder between the inlet opening and the outlet opening.

9. A smokeless cigarette, cigarillo, cigar or tobacco pipe comprising a base unit and at least one depot as claimed in one of claims 1 to 3 disposed in the base unit, wherein the substance is nicotine, a nicotine salt, a nicotine derivative or an ingredient of cigar or cigarette tobacco, which is adsorbed onto or bound into the nanopores.

10. The smokeless cigarette, cigarillo, cigar or tobacco pipe as claimed in claim 9, wherein the cigarette, cigarillo, cigar or tobacco pipe comprises a heat source to pre-heat the gas or to heat the depot.

11. A device adapted to release a substance into a stream of gas, comprising a depot as claimed in one of the claims 1 to 7 or a cartridge as claimed in claim 8, wherein the stream of gas can be conducted through the depot.

12. Use of a depot as claimed in one of the claims 1 to 7 or of a cartridge as claimed in claim 8, to release a substance into a volume of gas.

13. Use of a depot or cartridge as claimed in claim 12 in combination with a source of heat for pre-heating the gas or for heating the depot, in order to increase or to control the quantity of released substance.

## Revendications

1. Réservoir destiné à stocker une substance et à la délivrer dans une phase gazeuse, comportant une structure hybride constituée
- d'un squelette de support macroporeux à pores ouverts permettant le passage d'un gaz, et
- de particules nanoporeuses qui sont immobilisées sur le squelette de support et contiennent dans ses nanopores au moins une substance à libérer,
le squelette de support étant constitué de particules ou fibres liées les unes aux autres d'une manière durable.

2. Réservoir selon la revendication 1, les particules nanoporeuses étant constituées d'un autre matériau que le squelette de support.

3. Réservoir destiné à stocker une substance et à la délivrer dans une phase gazeuse, comportant une structure hybride constituée
- d'un squelette de support macroporeux à pores ouverts permettant le passage d'un gaz, et
- de nanopores formés dans le matériau du squelette de support,
le squelette de support étant sensiblement constitué d'un matériau inorganique tel que du verre, des silicates ou des alumosilicates, et les nanopores contenant au moins une substance à libérer.

4. Réservoir selon l'une des revendications 1 à 3, ladite substance étant un principe actif pharmaceutique ou un arôme, lequel est contenu dans les nanopores sous une forme adsorbée ou liée.

5. Réservoir selon l'une des revendications 1 à 3, ladite substance étant de la nicotine, un sel de nicotine ou un constituant du tabac de cigare ou de cigarette, lequel/laquelle est contenu(e) dans les nanopores sous une forme adsorbée ou liée.

6. Réservoir selon l'une des revendications 1 à 3, ladite substance étant
un insecticide, un acaricide ou un rodenticide, ou
un principe actif biologique tel que la cinerine, la jasmoline ou la pyréthrine, ou
un répulsif destiné à repousser des animaux domestiques ou sauvages, ou
un répulsif d'insectes tel que le p-menthanediol, le diéthyltoluamide ou la perméthrine
et étant contenue dans les nanopores sous une forme adsorbée ou liée.

7. Réservoir selon l'une des revendications 1 à 3, ladite substance étant un phéromone permettant d'attirer des insectes d'une manière ciblée.

8. Cartouche pour un dispositif de dosage, comprenant :
- un cylindre, ledit cylindre ayant au moins une entrée et au moins une sortie,
- un réservoir selon l'une des revendications 1 à 7, ledit réservoir étant maintenu au sein dudit cylindre entre l'entrée et la sortie.

9. Cigarette, cigarillo, cigare ou pipe à tabac sans fumée, comportant un corps de base et au moins un réservoir selon l'une des revendications 1 à 3 qui est disposé dans le corps de base, ladite substance étant de la nicotine, un sel de nicotine, un dérivé de nicotine ou un constituant du tabac de cigare ou de cigarette, lequel/laquelle est contenu(e) dans les nanopores sous une forme adsorbée ou liée.

10. Cigarette, cigarillo, cigare ou pipe à tabac sans fumée selon la revendication 9, ledit/ladite cigarette, cigarillo, cigare ou pipe à tabac comportant une source de chaleur permettant de préchauffer le gaz ou de chauffer ledit réservoir.

11. Dispositif adapté à la libération d'une substance dans un flux gazeux, comportant un réservoir selon l'une des revendications 1 à 7 ou une cartouche selon la revendication 8, ledit flux gazeux pouvant être passé à travers ledit réservoir.

12. Utilisation d'un réservoir selon l'une des revendications 1 à 7 ou d'une cartouche selon la revendication 8 pour délivrer une substance dans un volume de gaz.

13. Utilisation d'un réservoir ou d'une cartouche selon la revendication 12 en association avec une source de chaleur permettant de préchauffer le gaz ou de chauffer ledit réservoir, pour ainsi augmenter la quantité de substance délivrée ou l'ajuster de manière contrôlée.
